(19) Europäisches Patentamt
European Patent Office
Office européen des brevets

(11) EP 1 140 813 B1

(12) FASCICULE DE BREVET EUROPEEN

(45) Date de publication et mention de la délivrance du brevet:
**16.06.2004 Bulletin 2004/25**

(21) Numéro de dépôt: **99961161.9**

(22) Date de dépôt: **23.12.1999**

(51) Int Cl.$^7$: **C07C 319/14**, C07C 323/29

(86) Numéro de dépôt international:
**PCT/FR1999/003273**

(87) Numéro de publication internationale:
**WO 2000/039079 (06.07.2000 Gazette 2000/27)**

# (54) PROCEDE DE PREPARATION DE THIOETHERS AROMATIQUES DE TYPE DIPHENYLE

VERFAHREN ZUR HERSTELLUNG VON AROMATISCHEN THIOETHERN VOM DIPHENYLTYP

METHOD FOR PREPARING AROMATIC DIPHENYL THIOETHERS

(84) Etats contractants désignés:
**AT BE CH CY DE DK ES FI FR GB GR IE IT LI LU MC NL PT SE**

(30) Priorité: **23.12.1998 FR 9816372**

(43) Date de publication de la demande:
**10.10.2001 Bulletin 2001/41**

(73) Titulaire: **RHODIA CHIMIE**
**92408 Courbevoie Cédex (FR)**

(72) Inventeurs:
- **SCHLAMA, Thierry**
  **F-69570 Dardilly (FR)**
- **BIGOURAUX, Jean-Christophe**
  **F-42800 Dargoire (FR)**

(74) Mandataire: **Dutruc-Rosset, Marie-Claude et al**
**Rhodia Services,**
**Direction de la Propriété Industrielle,**
**40, rue de la Haie-Coq**
**93306 Aubervilliers Cedex (FR)**

(56) Documents cités:
- **CHEMICAL ABSTRACTS, vol. 56, no. 8, 16 avril 1962 (1962-04-16) Columbus, Ohio, US; abstract no. 8612i, A. ARCORIA, ET AL.: "Near ultraviolet spectra of p-phenoxyphenyl phenyl sulphone and of the corresponding methyl, chloro, nitro, amino and acetylamino derivatives" colonne 8612; XP002112736 -& A. ARCORIA, ET AL.: "Spettri nel vicino u.v. del p-(fenossi)-difenilsolfone e corrispondenti metil-, cloro-, nitro-, amino ad acetilamino-derivati" GAZZETTA CHIMICA ITALIANA, vol. 91, 1961, pages 223-241, XP002112733 Società Chimica Italiana, Rome, IT ISSN: 0016-5603**
- **U. SCHMIDT, ET AL.: "Über organische Schwefelradikale, III. Organische Schwefel-Radikalionen (Sulfiniumsalze)" LIEBIGS ANNALEN DER CHEMIE, vol. 672, 1964, pages 78-90, XP002112732 Verlag Chemie, Weinheim, DE ISSN: 0170-2041**
- **CHEMICAL ABSTRACTS, vol. 52, no. 22, 25 novembre 1958 (1958-11-25) Columbus, Ohio, US; abstract no. 19446h, A. MAGINI, ET AL.: "Some aryl sulphones" colonne 19446; XP002112737 -& A. MANGINI, ET AL.: "Su alcuni aril-sulfoni" BOLLETTINO SCIENTIFICO DELLA FACOLTA DI CHIMICA INDUSTRIALE DI BOLOGNA, vol. 14, 1956, pages 81-95, XP002112734 Lanichelli, Bologna, IT ISSN: 0366-3205**

EP 1 140 813 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

- CHEMICAL ABSTRACTS, vol. 51, no. 12, 25 juin 1957 (1957-06-25) Columbus, Ohio, US; abstract no. 8685i, G. LEANDRI, ET AL.: "Sulphides and disulphides, XVIII. Oxidation of p-phenylene disulphides" colonne 8685; XP002112738 -& G. LEANDRI, ET AL.: "Ricerche sui solfuri e disolfuri, XVIII. Ossidazione de p-fenilen-disolfuri" ANNALI DI CHIMICA, vol. 46, 1956, pages 1069-1079, XP002112735 Società Chimica Italiana, Rome, IT ISSN: 0003-4592

## Description

La présente invention a pour objet un procédé de préparation de thioéthers aromatiques de type diphényle.

Plus, précisément l'invention vise la préparation d'un composé aromatique comprenant un enchaînement d'au moins deux groupes phényle dont au moins l'un des deux porte un groupe thioéther.

L'invention vise plus particulièrement la préparation de 4-chloro-4'-thiométhyldiphényléther.

Lorsque l'on souhaite introduire un groupe fonctionnel sur une molécule de type diphényle, le problème réside dans le fait qu'il est difficile d'introduire un groupe fonctionnel sur seulement l'un des noyaux benzéniques.

Il est connu selon Chemical Abstracts 56(8), 1962 n°8612i, de préparer le composé 4-(2-$MeC_6H_4S$)$C_6H_4OPh$, par réaction du sel de diazonium p-$H_2NC_6H_4$-OPh.HCl et de o-$HSC_6H_4Me$ en présence de soude.

L'objectif de la présente invention est de fournir un autre procédé qui permette d'introduire au moins un groupe thioéther sur l'un des groupes phényle.

Il a maintenant été trouvé et c'est ce qui fait l'objet de la présente invention, un procédé de préparation d'un thioéther aromatique de type diphényle caractérisé par le fait que l'on fait réagir en milieu aqueux, un sel de diazonium d'un composé aromatique de type diphényle et un composé soufré de type disutfure, en présence d'une quantité efficace d'un catalyseur de couplage.

Par thioéther aromatique de type diphényle, on entend un enchaînement de deux groupes phényle reliés entre eux et dont au moins l'un des noyaux benzéniques portent une fonction thioéther.

Une variante préférée du procédé de l'invention consiste à préparer le thioéther selon un procédé qui enchaîne la préparation du sel de diazonium à partir de l'amine aromatique correspondante puis à effectuer, sans séparation, la réaction avec le composé soufré.

Conformément au procédé de l'invention, on peut partir d'une amine aromatique de type diphényle que l'on transforme dans une première étape en sel de diazonium.

Par amine aromatique de type diphényle, on entend un enchaînement de deux groupes phényle reliés entre eux et dont au moins l'un des noyaux benzéniques portent une fonction amine.

L'amine aromatique de départ peut être symbolisée par la formule générale (I) :

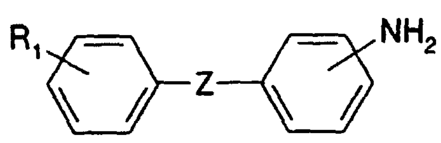

(I)

dans ladite formule (I) :

- $R_1$ représente un atome d'hydrogène ou un substituant R,
- Z représente :
  - . un lien valentiel
  - . un groupe alkylène ou alkylidène ayant de 1 à 4 atomes de carbone, de préférence, un groupe méthylène ou isopropylidène,
  - . un groupe B qui peut être un atome ou groupe suivant :
    -O-, -CO-, -COO-, -OOC-, -OCOO-
    -S-, -SO-, -$SO_2$-,

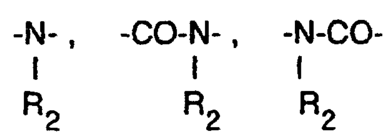

dans ces formules, $R_2$ représente un atome d'hydrogène ou un groupe alkyle ayant de 1 à 6 atomes de carbone ou phényle.

Dans la formule (I), l'un ou les deux cycles benzéniques peuvent être substitués ce qui signifie que dans le substrat de départ de type diphényle, au moins l'un des 5 atomes d'hydrogène du cycle aromatique peut être remplacé par un atome autre qu'un atome d'hydrogène. Il peut s'agir en particulier d'un atome d'halogène, de carbone, d'oxygène

ou d'azote.

Le groupe $R_1$, représente un atome d'hydrogène ou tout autre groupe R.

Le groupe R peut être d'une nature quelconque dans la mesure où il ne gêne pas la réaction de diazotation.

Des exemples de substituants sont donnés ci-dessous mais cette liste ne présente pas de caractère limitatif. On peut citer notamment :

- un groupe alkyle linéaire ou ramifié ayant de préférence de 1 à 6 atomes de carbone et encore plus préférentiellement de 1 à 4 atomes de carbone,
- un groupe alcényle linéaire ou ramifié ayant de préférence de 2 à 6 atomes de carbone et encore plus préférentiellement de 2 à 4 atomes de carbone,
- un groupe halogénoalkyle linéaire ou ramifié ayant de préférence de 1 à 4 atomes de carbone, et de 1 à 9 atomes d'halogène,
- un groupe cycloalkyle ayant de 3 à 7 atomes de carbone, de préférence, le groupe cyclohexyle,
- un groupe phényle,
- un groupe hydroxyle,
- un groupe $NO_2$,
- un groupe alkoxy $R_3$-O- ou thioéther $R_3$-S- dans lequel $R_3$ représente un groupe alkyle linéaire ou ramifié ayant de 1 à 6 atomes de carbone et, de préférence, de 1 à 4 atomes de carbone ou le groupe phényle,
- un groupe $-N-(R_2)_2$ dans lequel les groupes $R_2$ identiques ou différents, représentent un atome d'hydrogène, un groupe alkyle linéaire ou ramifié ayant de 1 à 6 atomes de carbone et, de préférence, de 1 à 4 atomes de carbone ou un groupe phényle,
- un groupe $-NH-CO-R_2$ dans lequel le groupe $R_2$ a la signification donnée précédemment,
- un groupe carboxy ou dérivé $R_2$-O-CO- dans lequel le groupe $R_2$ a la signification donnée précédemment,
- un groupe acyloxy ou aroyloxy $R_3$-CO-O- dans lequel le groupe $R_3$ a la signification donnée précédemment,
- un groupe $B(OR_3)_2$ dans lequel le groupe $R_3$ a la signification donnée précédemment,
- un atome d'halogène, de préférence, un atome de fluor,
- un groupe $CF_3$,
- deux groupes R peuvent être liés et former ensemble un groupe alkylènedioxy ayant de 1 à 4 atomes dans le groupe alkytène, de préférence, un groupe méthylènedioxy ou éthylènedioxy.

Comme groupes R préférés, on peut citer un atome d'halogène, de préférence, un atome de fluor, chlore ou brome ou un groupe halogénoalkyle, de préférence perfuoroafkyle ; un groupe hydroxyle : un groupe alkyle ou alkoxy ayant de 1 à 6 atomes de carbone, de préférence, de 1 à 4 atomes de carbone ; un groupe amino ou un groupe amino substitué par un ou deux groupes alkyle ayant de 1 à 6 atomes de carbone, de préférence, de 1 à 4 atomes de carbone.

Les composés mis en oeuvre préférentiellement sont ceux qui répondent à la formule (I) dans laquelle $R_1$ représente un atome de fluor ou de chlore et Z représente un atome d'oxygène.

Conformément au procédé de l'invention, on commence dans une première étape à préparer le sel de diazonium. de l'amine aromatique de type diphényle répondant préférentiellement à la formule (I).

A cet effet, pour transformer le groupe amino en groupe diazonium, on fait réagir le substrat de départ avec un acide. Bien que l'on puisse utiliser un acide tel que l'acide sulfurique, on préfère mettre en oeuvre un acide de type hydracide afin de salifier le groupe amine sous forme d'halohydrate.

Ainsi, préférentiellement, on fait réagir le substrat de départ répondant préférentiellement à la formule (1) et l'acide chlorhydrique ou bromhydrique.

La quantité d'acide mise en oeuvre est telle que le rapport molaire entre le nombre d'ions $H^+$ et le nombre de moles de substrat varie entre 2,0 et 2,5, de préférence entre 2,0 et 2,2.

Dans une étape suivante, on prépare le sel de diazonium par réaction de l'amine aromatique de type diphényle sous forme d'halohydrate avec un réactif de diazotation qui est toute source de $NO^+$.

Ainsi, on peut partir du dioxyde d'azote $NO_2$, de l'anhydride azoteux $N_2O_3$, du peroxyde d'azote $N_2O_4$, de l'oxyde d'azote NO associé à un agent oxydant tel que, par exemple, l'acide nitrique, le dioxyde d'azote ou l'oxygène. Dans le cas où le réactif est gazeux dans les conditions réactionnelles, on le fait buller dans le milieu.

On peut également faire appel à l'acide nitreux, à un sulfate de nitrosyle ou nitrose ou à un sel nitreux, de préférence un sel de métal alcalin, et encore plus préférentiellement, le sodium.

Il est également possible de mettre en oeuvre des nitrites d'alkyle et plus particulièrement ceux répondant à la formule (II) :

$$R_a\text{- ONO} \qquad \text{(II)}$$

dans ladite formule (II), $R_a$ représente un groupe alkyle linaire ou ramifié ayant de 1 à 12 atomes de carbone, de préférence, de 1 à 4 atomes de carbone.

On choisit avantageusement le nitrite de sodium.

La quantité de réactif de diazotation mise en oeuvre peut varier largement. Lorsqu'elle est exprimée par le rapport molaire amine aromatique/réactif de diazotation défini en $NO^+$, elle est au moins égale à la quantité stoechiométrique mais il est préférable qu'elle soit mise en oeuvre en un excès pouvant atteindre 120 % de la quantité stoechiométrique, et de préférence, compris entre 100 % et 120 %.

En ce qui concerne la concentration du substrat amine aromatique dans le milieu réactionnel, elle est de préférence comprise entre 0,5 et 2,5 mol/l et se situe préférentiellement aux environs de 1 mol/l.

La préparation de l'halohydrate de l'amine se fait par simple mélange de l'amine de départ et de l'acide.

La réaction est avantageusement effectuée à une température se situant entre 50°C et 100°C.

On ajoute ensuite le réactif de diazotation, de préférence progressivement par fractions ou en continu.

Quant à la température de la réaction de diazotation, celle-ci est généralement conduite à basse température se situant avantageusement entre -10°C et 20°C, de préférence entre 0 et 10°C.

Conformément au procédé de l'invention, on fait réagir avec le composé soufré, le sel de diazonium obtenu, répondant préférentiellement à la formule (III).

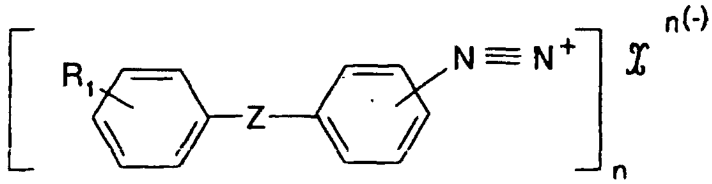

(III)

dans ladite formule (III) :

- $\mathcal{X}$ représente un atome d'halogène X, de préférence un atome de chlore ou de brome, un groupe $HSO_4$-, un groupe $SO_4^=$,
- $R_1$ et Z ont la signification donnée précédemment,
- n est égal à 1 ou 2.

Le composé soufré mis à réagir répond de préférence, à la formule (IV) suivante :

$$R_4\text{-S-S-}R_5 \qquad \text{(IV)}$$

dans ladite formule (IV) :

- $R_4$ et $R_5$, identiques ou différents, représentent un groupe hydrocarboné ayant de 1 à 24 atomes de carbone, qui peut être un groupe aliphatique acyclique saturé ou insaturé, linéaire ou ramifié ; un groupe carbocyclique ou hétérocyclique, saturé, insaturé ou aromatique, monocyclique ou polycyclique ; un groupe aliphatique saturé ou insaturé, linéaire ou ramifié, porteur d'un substituant cyclique.

Le composé soufré qui intervient dans le procédé de l'invention répond à la formule (IV) dans laquelle $R_4$ et $R_5$ peuvent prendre diverses significations. Différents exemples sont donnés ci-après mais ils ne sont en aucun cas limitatif.

Dans les composés de formule (IV), $R_4$ et $R_5$ représentent préférentiellement un groupe aliphatique acyclique, saturé ou insaturé, linéaire ou ramifié ayant de préférence de 1 à 24 atomes de carbone, saturé ou comprenant une à plusieurs insaturations sur la chaîne, généralement, 1 à 3 insaturations qui peuvent être des doubles liaisons simples ou conjuguées ou des triples liaisons.

$R_4$ et $R_5$ représentent plus particulièrement un groupe alkyle, alcényle, alcadiényle, linéaire ou ramifié ayant de préférence de 1 à 12 atomes de carbone.

$R_4$ et $R_5$ représentent un groupe halogénoalkyle linéaire ou ramifié ayant de préférence de 1 à 12 atomes de carbone et encore plus préférentiellement de 1 à 4 atomes de carbone, et de 3 à 25 atomes d'halogène,

La chaîne hydrocarbonée peut être éventuellement :

- interrompue par un atome ou groupe fonctionnel et l'on peut citer les groupes B précités,
- et/ou porteuse de l'un des substituants suivants :
  $-OH, -COR_3, -COOR_2, -CHO, -CN, -NO_2, -X, -CF_3$

dans ces formules, les groupes $R_2$, identiques ou différents et le groupe $R_3$ ayant la signification donnée précédemment.

Les groupes $R_4$ et $R_5$ peuvent représenter un groupe halogénoalkyle, de préférence perhalogénoalkyle ou un groupe halogénoalcényle.

Dans la formule (IV), le groupe aliphatique acyclique, saturé ou insaturé, linéaire ou ramifié peut être éventuellement porteur d'un substituant cyclique. Par cycle, on entend un cycle carbocyclique ou hétérocyclique, saturé, insaturé ou aromatique.

Le groupe aliphatique acyclique peut être relié au cycle par un lien valentiel ou par l'un des groupes B tels que précités.

Comme exemples de substituants cycliques, on peut envisager des substituants cycloaliphatiques, aromatiques ou hétérocycliques, notamment cycloaliphatiques comprenant 6 atomes de carbone dans le cycle ou benzéniques, ces substituants cycliques étant eux-mêmes éventuellement porteurs d'un ou plusieurs substituants.

Comme exemples de tels groupes, on peut mentionner, entre autres, le groupe benzyle.

Dans la formule générale (IV), $R_4$ et $R_5$ peuvent représenter un groupe carbocyclique, monocyclique. Le nombre d'atomes de carbone dans le cycle peut varier largement de 3 à 8 atomes de carbone mais il est de préférence égal à 5 ou 6 atomes de carbone.

Le carbocycle peut être saturé ou comprenant 1 ou 2 insaturations dans le cycle, de préférence de 1 à 2 doubles liaisons.

Comme exemples préférés de groupes $R_4$ et $R_5$, on peut citer les groupes cyclohexyle ou cyclohexène-yle.

Dans le cas où $R_4$ et $R_5$ représentent un groupe carbocyclique, monocyclique, saturé ou insaturé, il est possible que l'un ou plusieurs des atomes du carbone du cycle soient remplacés par un hétéroatome, de préférence, oxygène, azote ou soufre ou par un groupe fonctionnel, de préférence carbonyle ou ester, conduisant ainsi à un composé hétérocyclique, monocyclique. Le nombre d'atomes dans le cycle peut varier largement de 3 à 8 atomes mais il est de préférence égal à 5 ou 6 atomes.

Les groupes $R_4$ et $R_5$ peuvent être également carbocyclique, polycyclique, de préférence bicyclique ce qui signifie qu'au moins deux cycles ont deux atomes de carbone en commun. Dans le cas des groupes polycycliques, le nombre d'atomes de carbone dans chaque cycle varie entre 3 et 6 : le nombre total d'atomes de carbone étant égal de préférence à 7.

Les groupes $R_4$ et $R_5$ peuvent être également hétérocyclique, polycyclique, de préférence bicyclique ce qui signifie qu'au moins deux cycles ont deux atomes en commun. Dans ce cas, le nombre d'atomes dans chaque cycle varie entre 3 et 6 et est plus préférentiellement égal à 5 ou 6.

Les groupes $R_4$ et $R_5$ peuvent représenter préférentiellement un groupe carbocyclique aromatique, et notamment benzénique ou un enchaînement de 2 ou 3 noyaux benzéniques séparés par des atomes ou groupes B tels que définis.

Comme exemples de groupes $R_4$ et $R_5$ répondant à la formule (IV), on peut mentionner plus précisément les groupes phényle.

$R_4$ et $R_5$ peuvent également représenter un groupe hydrocarboné aromatique polycyclique ; les cycles pouvant former entre eux des systèmes ortho-condensés, ortho- et péri-condensés. On peut citer plus particulièrement, le groupe naphtyle.

Dans la formule générale (IV), $R_4$ et $R_5$ peuvent également représenter un groupe hétérocyclique aromatique, comportant notamment 5 ou 6 atomes dans le cycle dont 1 ou 2 hétéroatomes tels que les atomes d'azote, de soufre et d'oxygène.

A titre illustratif de groupes hétérocycliques, on peut citer les groupes tétrahydrofuryle, tétrahydrothiényle, pyrrolidinyle, furyle, thiényle, pyrrolyle, pyridyle.

$R_4$ et $R_5$ peuvent aussi représenter un groupe hétérocyclique aromatique polycyclique défini comme étant soit un groupe constitué par au moins 2 hétérocycles aromatiques ou non contenant au moins un hétéroatome dans chaque cycle et formant entre eux des systèmes ortho- ou ortho- et péricondensés ou soit un groupe constitué par au moins un cycle hydrocarboné aromatique ou non et au moins un hétérocycle aromatique ou non formant entre eux des systèmes ortho- ou ortho- et péricondensés.

On donne ci-après, à titre illustratif des exemples des groupes aromatiques polycycliques : les groupes isoquinolyle, quinolyle, naphtyridinyle, benzofurannyle, indolyle.

Il est à noter que si le groupe $R_4$ et $R_5$ comprend un cycle quelconque, il est possible que ce cycle porte un substituant. La nature du substituant est quelconque dans la mesure où il n'interfère pas au niveau du produit désiré. Les substituants sont de même nature que R.

Comme exemples préférés de groupes $R_4$, $R_5$, on peut citer les groupes alkyle linéaires ou ramifiés, ayant

de 1 à 4 atomes de carbone, 2-carboxyéthyle, cyclohexyle, phényle, benzyle, benzoyle, pyridyle..

Le procédé est mis en oeuvre aisément avec de nombreux composés soufrés.

On donne des exemples préférés de composés soufrés de type disutfure, on peut mentionner notamment :

- le diméthyldisuffure,
- le diéthyldisulfure,
- le di-n-propyldisulfure,
- le diisopropyldisuffure,
- le di-n-butyldisulfure,
- le diisobutyldisutfure,
- le di-sec-butyldisulfure,
- le di-tert-butyldisutfure,
- le diisoamyldisulfure,
- le di-n-hexyldisulfure,
- le di-tert-heptyldisuffure,
- le di-n-undécyldisulfure,
- le distéaryldisulfure,
- le diallyldisulfure,
- le dicyclohexyldisulfure,
- le diphényldisulfure,
- le dibenzyldisulfure,
- le dibenzoyldisulfure,
- le dithiopyridine,
- l'acide dithioglycolique.

Parmi les composés précités, on préfère mettre en oeuvre dans le procédé de l'invention, les dialkyldisulfures ayant de préférence de 1 à 4 atomes de carbone dans la partie alkyle.

La quantité de composé soufré est telle que le rapport entre le nombre de composé soufré et le nombre de moles de sel de diazonium varie entre 1 et 1,5.

La réaction de couplage est conduite en milieu aqueux. La quantité d'eau présente dans le milieu réactionnel représente généralement de 100 à 500 % du poids de l'amine aromatique.

Une variante du procédé de l'invention consiste à ajouter un solvant organique qui est choisi inerte dans les conditions réactionnelles.

Comme exemples de solvants organiques, on peut citer les acides monocarboxyliques aliphatiques saturés et plus particulièrement, l'acide formique, l'acide acétique, l'acide propionique, l'acide butyrique, l'acide isobutyrique, l'acide pentanoïque, l'acide 2-méthylbutanoïque.

Parmi tous les acides monocarboxylique aliphatiques saturés, l'acide acétique est choisi préférentiellement.

On peut envisager également de faire appel à un solvant tel que l'acétone ou le diméthylformamide.

La quantité de solvant organique mise en oeuvre exprimée par rapport au poids de l'amine de départ varie avantageusement entre 100 % et 1000 %, de préférence, entre 200 % et 500 %.

Conformément au procédé de l'invention, on conduit la réaction du sel de diazonium répondant préférentiellement à la formule (III) avec le composé soufré répondant préférentiellement à la formule (IV) : la réaction étant alors conduite en présence d'un catalyseur de couplage.

Le catalyseur de couplage est un catalyseur comprenant au moins un élément métallique choisi parmi la 4$^{ème}$ et 5$^{ème}$ période des groupes IIIA, IVA, VA, VIA, VIIA, VIII, IB et IIB de la classification périodique des éléments.

On peut citer préférentiellement, les éléments métalliques suivants ; cuivre, fer, cobalt, nickel, palladium, platine.

Pour la définition des éléments, on se réfère ci-après à la Classification périodique des éléments publiée dans le Bulletin de la Société Chimique de France, n°1 (1966).

Ainsi, les éléments métalliques peuvent être également apportés sous forme de métal zéro ou d'un dérivé inorganique tel qu'un oxyde ou un hydroxyde. Il est possible de faire appel à un sel minéral de préférence, nitrate, sulfate, oxysulfate, halogénure, oxyhalogénure, silicate, carbonate, ou à un dérivé organique de préférence, cyanure, oxalate, acétylacétonate ; alcoolate et encore plus préférentiellement méthylate ou éthylate ; carboxylate et encore plus préférentiellement acétate. Peuvent être également mis en oeuvre des complexes, notamment chlorés ou cyanés desdits métaux et/ou de métaux alcalins, de préférence sodium, potassium ou d'ammonium

Comme exemples plus spécifiques de catalyseurs au palladium, on peut citer notamment le chlorure de palladium (II), le nitrate de palladium (II) hydraté, le sulfate de palladium (II) dihydraté, l'acétate de palladium (II), le tétrachloropalladate(II) d'ammonium, l'hexachloropalladate (IV) de potassium, le tétrakistriphénylphosphine de palladium

(II).

Pour ce qui est des catalyseurs au platine, on peut mentionner entre autres, le chlorure de platine (II), le tétrachloroplatinate (II) d'ammonium. l'hexachloroplatinate (IV) d'ammonium, le tétrachloroplatinate (IV) de sodium hydraté, l'hexachloroplatinate (IV) de sodium hexahydraté, l'hexachloroplatinate (IV) de potassium, l'acide chloroplatinique hexahydraté.

Pour ce qui est des catalyseurs au nickel ou cobalt, on peut citer notamment le chlorure ou bromure de nickel (II), le chlorure ou le bromure de cobalt (II).

Le catalyseur de choix mis en oeuvre dans le procédé de l'invention est à base de cuivre.

Comme exemples de catalyseurs susceptibles d'être mis en oeuvre, on peut citer le cuivre métal ou les composés organiques ou inorganiques du cuivre I ou du cuivre II.

Les catalyseurs à base de cuivre 0 et I sont préférés.

A titre non limitatif, on peut citer comme composés du cuivre, le bromure cuivreux, le bromure cuivrique, l'iodure cuivreux, le chlorure cuivreux, le chlorure cuivrique, le carbonate de cuivre II basique, le nitrate cuivreux, le nitrate cuivrique, le sulfate cuivreux, le sulfate cuivrique, le sulfite cuivreux, l'oxyde cuivreux, l'acétate cuivreux, l'acétate cuivrique, le trifluorométhylsulfonate cuivrique, l'hydroxyde cuivrique, le méthylate de cuivre I, le méthylate de cuivre II, le méthylate chlorocuivrique de formule $ClCuOCH_3$.

La quantité de catalyseur mise en oeuvre exprimée par rapport au poids de sel de diazonium varie généralement entre 0,1 % et 20 % en mol, de préférence, entre 1 et 10 %.

La réaction de couplage entre le sel de diazonium répondant préférentiellement à la formule (III) et le composé soufré est avantageusement conduite à une température variant entre 0°C et 120°C, de préférence, entre 80°C et 100°C.

Généralement, la réaction est conduite à pression atmosphérique mais des pressions plus faibles ou plus élevées peuvent également convenir. On travaille sous pression autogène lorsque la température de réaction est supérieure à la température d'ébullition des réactifs et/ou des produits.

Selon une variante préférée du procédé de l'invention, on conduit le procédé de l'invention, sous atmosphère contrôlée de gaz inertes. On peut établir une atmosphère de gaz rares, de préférence l'argon mais il est plus économique de faire appel à l'azote.

On poursuit la réaction jusqu'à transformation complète du sel de diazonium, l'évolution de la réaction pouvant être suivie à l'aide d'un moyen analytique classique tel que chromatographie en phase gazeuse ou chromatographie liquide haute performance.

La durée de la réaction est généralement courte de l'ordre de 30 min à 2 heures.

D'un point de vue pratique, on met les deux réactifs en présence dans un ordre quelconque. Selon une variante préférée, on ajoute préférentiellement le composé soufré dans le sel de diazonium puis le catalyseur.

Ainsi, en fin de réaction, on obtient deux phases, l'une aqueuse comprenant tous les sels formés et une phase organique comprenant en plus des éventuels réactifs excédentaires, le composé attendu qui répond préférentiellement à la formule (VI):

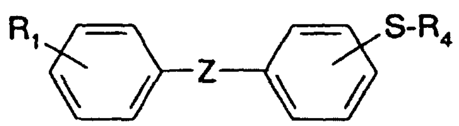

(VI)

dans ladite formule (VI), $R_1$, $R_4$, et Z ont la signification donnée précédemment.

On récupère le produit désiré à partir de la phase organique selon les techniques classiques utilisées. A titre illustratif, on précise que l'on peut ajouter un solvant organique, par exemple l'éther isopropylique ou un alcane tel que le méthylcyclohexane afin d'extraire tous les composés organiques et ensuite, à partir de cette phase organique, séparer le composé selon les techniques usuelles de séparation comme notamment la distillation ou la cristallisation dans un solvant approprié, de préférence, un alcool et plus particulièrement le méthanol ou l'isopropanol.

On donne ci-après des exemples de réalisation de l'invention donné à titre illustratif et sans caractère limitatif.

Dans les exemples, les abréviations suivantes signifient :

$$TT= \frac{\text{nombre de moles de 4-chloro-4'-aminodiphényléther transformées}}{\text{nombre de moles de 4-chloro-4'-aminodiphényléther introduites}} \%$$

$$RR = \frac{\text{nombre de moles de 4-chloro-4'-thiométhyldiphényléther formées}}{\text{nombre de moles de 4-chloro-4'-aminodiphényléther introduites}} \%$$

Exemple 1

Dans un réacteur de 50 ml à double enveloppe, on charge 2,20 g (10 mmol) de 4-chloro-4'-aminodiphényléther, 8 ml d'eau, et sous agitation, 2,33 g (23 mmol) d'acide chlorhydrique à 36 %.
On chauffe à 90 °C et le mélange devient homogène.
On maintient cette température pendant 45 min puis l'on refroidit à 10°C.
On coule 0,69 g (10 mmol) d'une solution aqueuse de nitrite de sodium à 30 %, en deux heures à l'aide d'un pousse seringue.
Dans un réacteur de 100 ml, l'on ajoute à 50°C le sel de diazonium, goutte à goutte, dans un mélange de 0,82 g de diméthyldisulfure et 5 ml d'eau, et 31 mg de cuivre métallique.
On ajoute 15 ml d'éther isopropylique.
On vide le réacteur et on lave la phase organique avec 100 ml d'eau et 100 ml de bisulfite de sodium à 10 % dans l'eau et à nouveau 100 ml d'eau.
On concentre la phase organique sous pression réduite de 2 mbar, à 50°C, pendant 1 heure.
On obtient 1,38 g de 4-chloro-4'-thiométhyldiphényléther ce qui correspond à un taux de transformation de 100 % et un rendement RR de 55 %.
On peut purifier le produit obtenu par cristallisation dans le méthanol.

Exemples 2 à 14

On répète l'exemple précédent mais en changeant la nature du catalyseur.
On détermine la quantité de produit formé par dosage par chromatographie en phase gazeuse.
Les résultats sont consignés dans le tableau (1).

Tableau (I)

| Ref ex | Nature du catalyseur | Rendement |
|---|---|---|
| 2 | $CuBr_2$ | 63,0% |
| 3 | $CuSO_4$ | 62,7% |
| 4 | $CuCl_2$, 2 $H_2O$ | 61,2 % |
| 5 | CuBr | 48,9% |
| 6 | $Cu_2O$ | 37,3% |
| 7 | $Pd(AcO)_2$ | 26,6 % |
| 8 | $Pd(PPh_3)_4$ | 25,5 % |
| 9 | $NiBr_2$ | 24,6 % |
| 10 | $PdCl_2$ | 24,4% |
| 11 | $CoCl_2$, 6 $H_2O$ | 20,2% |
| 12 | Pd/C | 18,9% |
| 13 | $MnCl_2$ | 14,4% |
| 14 | $A_gNO_3$ | 10,9% |

Exemple 15

Dans un ballon tricol de 2,0 litres muni d'un thermomètre, d'une ampoule de coulée, d'un réfrigérant surmonté d'une réserve d'argon (baudruche) et maintenu sous atmosphère inerte et sous forte agitation magnétique,
165 g de Cl-Ph-O-Ph-$NH_2$ (0,75 mol) sont chargés. Par la suite, puis 300 ml d'acide acétique sont ajoutés.
L'ensemble est chauffé à 80°C puis 82,5 g d'une solution aqueuse d'acide chlorydrique concentré (37 %) sont additionnés lentement.
Le milieu réactionnel est agité environ 45 min à 80°C.
La température est laissée revenir à 65°C sous agitation.
Puis 2,13 g de $CuCl_2$ sont ajoutés dans le milieu réactionnel suivi de 132 ml de $Me_2S_2$.
Ensuite, une solution aqueuse de $NaNO_2$ (solution de 54 g dans 120 ml de $H_20$) est additionnée par une

ampoule de coulée.

Lorsque le dégagement gazeux cesse, la température est ramenée à l'ambiante et le milieu réactionnel est dilué avec 360 ml d'eau. Il apparait deux phases.

On ajoute 500 ml de méthylcyclohexane et on sépare les deux phases.

La phase aqueuse est à nouveau extraite par du méthylcyclohexane (500 ml).

Après évaporation du méthylcyclohexane, 242 g d'huile brune sont récupérés. Le produit brut est ensuite recristallisé pour donner 124 g de cristaux blanc rosé (rendement : 66 %, pureté : 97,2 % déterminé par chromatographie en phase gazeuse et Pf = 56,0°C).

Il est possible d'effectuer une deuxième recristallisation.

## Revendications

1. Procédé de préparation d'un thioéther aromatique de type diphényle **caractérisé par le fait que** l'on fait réagir en milieu aqueux, un sel de diazonium d'un composé aromatique de type diphényle et un composé soufré de type disulfure, en présence d'une quantité efficace d'un catalyseur de couplage.

2. Procédé selon la revendication 1 **caractérisé par le fait qu'**il consiste à préparer le sel de diazonium à partir de l'amine aromatique correspondante puis à effectuer, sans séparation, la réaction avec le composé soufré.

3. Procédé selon l'une des revendications 1 et 2 **caractérisé par le fait que** l'amine aromatique de départ répond à la formule générale (I) :

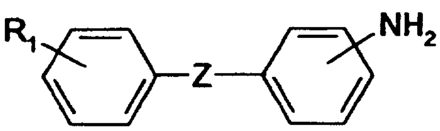

**(I)**

dans ladite formule (I) :

- $R_1$ représente un atome d'hydrogène ou un substituant R choisi parmi :
  - un groupe alkyle linéaire ou ramifié ayant de 1 à 6 atomes de carbone,
  - un groupe alcényle linéaire ou ramifié ayant de 2 à 6 atomes de carbone,
  - un groupe halogénoalkyle linéaire ou ramifié ayant de préférence de 1 à 4 atomes de carbone, et de 1 à 9 atomes d'halogène,
  - un groupe cycloalkyle ayant de 3 à 7 atomes de carbone,
  - un groupe phényle,
  - un groupe hydroxyle,
  - un groupe $NO_2$,
  - un groupe alkoxy $R_3$-O- ou thioéther $R_3$-S- dans lequel $R_3$ représente un groupe alkyle linéaire ou ramifié ayant de 1 à 6 atomes de carbone ou le groupe phényle,
  - un groupe -N-$(R_2)_2$ dans lequel les groupes $R_2$ identiques ou différents, représentent un atome d'hydrogène, un groupe alkyle linéaire ou ramifié ayant de 1 à 6 atomes de carbone ou le groupe phényle,
  - un groupe -NH-CO-$R_2$ dans lequel le groupe $R_2$ a la signification donnée précédemment,
  - un groupe carboxy ou dérivé $R_2$-O-CO- dans lequel le groupe $R_2$ a la signification donnée précédemment,
  - un groupe acyloxy ou aroyloxy $R_3$CO-O- dans lequel le groupe $R_3$ a la signification donnée précédemment,
  - un groupe $B(OR_3)_2$ dans lequel le groupe $R_3$ a la signification donnée précédemment,
  - un atome d'halogène,
  - un groupe $CF_3$,
  - deux groupes R peuvent être liés et former ensemble un groupe alkylènedioxy ayant de 1 à 4 atomes dans le groupe alkylène,
- Z représente :
  - . un lien valentiel

- . un groupe alkylène ou alkylidène ayant de 1 à 4 atomes de carbone,
- . un groupe B qui peut être un atome ou groupe suivant :
  -O- , -CO-, -COO-, -OOC-, -OCOO-
  -S-, -SO-, $-SO_2-$,

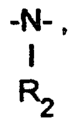 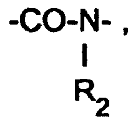 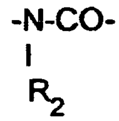

dans ces formules, $R_2$ représente un atome d'hydrogène ou un groupe alkyle ayant de 1 à 6 atomes de carbone ou phényle.

**4.** Procédé selon la revendication 3 **caractérisé par le fait que** l'amine aromatique de départ répond à la formule générale (I) dans laquelle

- $R_1$ représente un atome d'hydrogène ou un substituant R choisi parmi :

  - un groupe alkyle linéaire ou ramifié ayant de préférence de 1 à 4 atomes de carbone,
  - un groupe alcényle linéaire ou ramifié ayant de 2 à 4 atomes de carbone,
  - un groupe cyclohexyle,
  - un groupe alkoxy $R_3$-O- ou thioéther $R_3$-S- dans lequel $R_3$ représente un groupe alkyle linéaire ou ramifié ayant de 1 à 4 atomes de carbone ou le groupe phényle,
  - un groupe $-N-(R_2)_2$ dans lequel les groupes $R_2$ identiques ou différents, représentent un atome d'hydrogène, un groupe alkyle linéaire ou ramifié ayant de 1 à 4 atomes de carbone ou un groupe phényle,
  - un groupe -NH-CO-$R_2$ dans lequel le groupe $R_2$ a la signification donnée précédemment,
  - un groupe carboxy ou dérivé $R_2$-O-CO- dans lequel le groupe $R_2$ a la signification donnée précédemment,
  - un groupe acyloxy ou aroyloxy $R_3$CO-O- dans lequel le groupe $R_3$ a la signification donnée précédemment,
  - un groupe $B(OR_3)_2$ dans lequel le groupe $R_3$ a la signification donnée précédemment,
  - un atome de fluor,
  - deux groupes R peuvent être liés et former ensemble un groupe méthylènedioxy ou éthylènedioxy.

- Z représente :

  - . un lien valentiel
  - . un groupe méthylène ou isopropylidène,
  - . un groupe B qui peut être un atome ou groupe suivant :
    -O-, -CO- , -COO-, -OOC-, -OCOO-
    -S- , -SO-, $-SO_2-$,

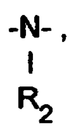 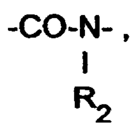 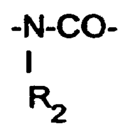

dans ces formules, $R_2$ représente un atome d'hydrogène ou un groupe alkyle ayant de 1 à 6 atomes de carbone ou phényle.

**5.** Procédé selon la revendication 4 **caractérisé par le fait que** l'amine aromatique de départ répond à la formule générale (1) dans laquelle le groupe R est un atome de fluor, chlore ou brome ou un groupe perfuoroalkyle ; un groupe hydroxyle : un groupe alkyle ou alkoxy ayant de 1 à 4 atomes de carbone ; un groupe amino ou un groupe amino substitué par un ou deux groupes alkyle ayant de 1 à 4 atomes de carbone.

**6.** Procédé selon l'une des revendications 1 à 5 **caractérisé par le fait que** l'amine aromatique de départ répond à la formule générale (I) dans laquelle $R_1$ représente un atome de fluor ou de chlore et Z représente un atome d'oxygène.

**7.** Procédé selon l'une des revendications 1 à 6 **caractérisé par le fait que** l'on prépare l'halohydrate de l'amine aromatique de type diphényle répondant préférentiellement à la formule (1) en faisant réagir cette dernière avec un hydracide, de préférence, l'acide chlorhydrique ou bromhydrique.

**8.** Procédé selon la revendication 7 **caractérisé par le fait que** que la quantité d'acide mise en oeuvre est telle que le rapport molaire entre le nombre d'ions $H^+$ et le nombre de moles de substrat varie entre 2,0 et 2,5, de préférence entre 2,0 et 2,2.

**9.** Procédé selon l'une des revendications 7 et 8 **caractérisé par le fait que** la réaction entre l'amine aromatique et l'hydracide est effectuée à une température se situant entre 50°C et 100°C.

**10.** Procédé selon l'une des revendications 1 à 9 **caractérisé par le fait que** l'on prépare le sel de diazonium par réaction de l'amine aromatique de type diphényle sous forme d'halohydrate avec un réactif de diazotation qui est toute source de $NO^+$.

**11.** Procédé selon la revendication 10 **caractérisé par le fait que** le réactif de diazotation est toute source de $NO^+$, de préférence l'oxyde d'azote NO associé à un agent oxydant : le dioxyde d'azote $NO_2$ ; l'anhydride azoteux $N_2O_3$; le peroxyde d'azote $N_2O_4$; l'acide nitreux ; le sulfate de nitrosyle ou un sel nitreux, de préférence un sel de métal alcalin, de préférence, le sodium ; un nitrite d'alkyle.

**12.** Procédé selon la revendications 11 **caractérisé par le fait que** la quantité de réactif de diazotation mise en oeuvre, exprimée par le rapport molaire amine aromatique/réactif de diazotation défini en $NO^+$, est comprise entre 100 % et 120 %.

**13.** Procédé selon l'une des revendications 10 à 12 **caractérisé par le fait que** la température de la réaction de diazotation est comprise entre -10°C et 20°C, de préférence entre 0 et 10°C.

**14.** Procédé selon l'une des revendications 10 à 13 **caractérisé par le fait que** le sel de diazonium obtenu, répond préférentiellement à la formule (III) :

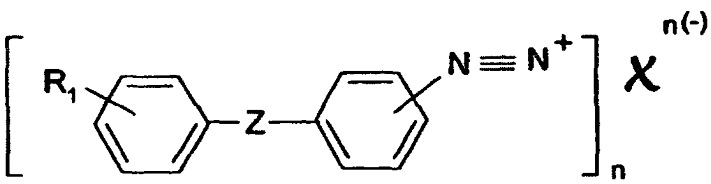

(III)

dans ladite formule (III) :

- X représente un atome d'halogène X, de préférence un atome de chlore ou de brome, un groupe $HSO_4^-$, un groupe $SO_4^=$,
- $R_1$ et Z ont la signification donnée précédemment dans l'une des revendications 3 à 6,
- n est égal à 1 ou 2.

**15.** Procédé selon l'une des revendications 1 et 2 **caractérisé par le fait que** le composé soufré mis à réagir répond à la formule (IV) suivante :

$$R_4\text{-S-S-}R_5 \qquad (IV)$$

dans ladite formule (IV) :

- $R_4$ et $R_5$, identiques ou différents, représentent un groupe hydrocarboné ayant de 1 à 24 atomes de carbone, qui peut être un groupe aliphatique acyclique saturé ou insaturé, linéaire ou ramifié ; un groupe carbocyclique ou hétérocyclique, saturé, insaturé ou aromatique, monocyclique ou polycyclique; un groupe aliphatique saturé ou insaturé, linéaire ou ramifié, porteur d'un substituant cyclique.

**16.** Procédé selon la revendication 15 **caractérisé par le fait que** le composé soufré répond à la formule générale (IV) dans $R_4$ et $R_5$ représentent un groupe aliphatique acyclique, saturé ou insaturé, linéaire ou ramifié ayant de préférence de 1 à 24 atomes de carbone, saturé ou comprenant une à plusieurs insaturations sur la chaîne, généralement, 1 à 3 insaturations qui peuvent être des doubles liaisons simples ou conjuguées ou des triples liaisons.

**17.** Procédé selon l'une des revendications 15 et 16 **caractérisé par le fait que** le composé soufré répond à la formule générale (IV) dans laquelle $R_4$ et $R_5$ représentent un groupe alkyle, alcényle, alcadiényle, linéaire ou ramifié ayant de préférence de 1 à 12 atomes de carbone : la chaîne hydrocarbonée pouvant être éventuellement :

- interrompue par un atome ou groupe fonctionnel B tel que défini dans la revendication 3,
- et/ou porteuse de l'un des substituants suivants :
  $-OH$, $-COR_3$, $-COOR_2$, $-CHO$, $-CN$, $-NO_2$, $-X$, $-CF_3$

dans ces formules, les groupes $R_2$, identiques ou différents et le groupe $R_3$ ayant la signification donnée précédemment.

**18.** Procédé selon l'une des revendications 15 à 17 **caractérisé par le fait que** le composé soufré répond à la formule générale (IV) dans laquelle $R_4$ et $R_5$ représentent un groupe halogénoalkyle linéaire ou ramifié ayant de préférence de 1 à 12 atomes de carbone et encore plus préférentiellement de 1 à 4 atomes de carbone, et de 3 à 25 atomes d'halogène,

**19.** Procédé selon l'une des revendications 15 à 18 **caractérisé par le fait que** le composé soufré répond à la formule générale (IV) dans laquelle $R_4$ et $R_5$ représentent un groupe aliphatique acyclique, saturé ou insaturé, linéaire ou ramifié porteur d'un substituant cyclique, de préférence, un cycle benzénique : le groupe aliphatique acyclique pouvant être relié au cycle par un lien valentiel ou par l'un des groupes B tels que définis dans la revendication 3.

**20.** Procédé selon la revendication 15 **caractérisé par le fait que** le composé soufré répond à la formule générale (IV) dans laquelle $R_4$ et $R_5$ représentent :

- un groupe carbocyclique monocyclique, saturé ou comprenant 1 ou 2 insaturations dans le cycle ; le nombre d'atomes de carbone dans le cycle varie de 3 à 8 atomes de carbone et est de préférence égal à 5 ou 6,
- un groupe carbocyclique polycyclique, de préférence bicyclique; le nombre d'atomes de carbone dans chaque cycle varie entre 3 et 6 : le nombre total d'atomes de carbone étant égal de préférence à 7.

**21.** Procédé selon la revendication 15 **caractérisé par le fait que** le composé soufré répond à la formule générale (IV) dans laquelle les groupes $R_4$ et $R_5$ représentent un groupe carbocyclique aromatique, et notamment benzénique ou un enchaînement de 2 ou 3 noyaux benzéniques séparés par des atomes ou groupes B tels que définis dans la revendication 3.

**22.** Procédé selon la revendication 15 **caractérisé par le fait que** le composé soufré répond à la formule générale (IV) dans laquelle $R_4$ et $R_5$ représentent :

- un groupe hétérocyclique aromatique comportant 5 ou 6 atomes dans le cycle dont 1 ou 2 hétéroatomes,
- un groupe carbocyclique ou hétérocyclique aromatique polycyclique.

**23.** Procédé selon l'une des revendications 15 à 22 **caractérisé par le fait que** le composé soufré répond à la formule générale (IV) dans laquelle $R_4$ et $R_5$, représentent un groupe alkyle linéaire ou ramifié, ayant de 1 à 4 atomes de carbone, un groupe 2-carboxyéthyle, cyclohexyle, phényle, benzyle, benzoyle, pyridyle.

**24.** Procédé selon la revendication 15 à 23 **caractérisé par le fait que** le composé soufré répondant à la formule générale (IV) dans laquelle $R_4$ et $R_5$ sont identiques.

**25.** Procédé selon la revendication 15 **caractérisé par le fait que** le composé soufré répondant à la formule générale (IV) est un composé de type disulfure choisi parmi :

- le diméthyldisulfure,
- le diéthyldisulfure,

- le di-n-propyldisulfure,
- le diisopropyldisulfure,
- le di-n-butyldisulfure,
- le diisobutyldisulfure,
- le di-sec-butyldisulfure,
- le di-tert-butyldisulfure,
- le diisoamyldisulfure,
- le di-n-hexyldisulfure,
- le di-tert-heptyldisulfure,
- le di-n-undécyldisulfure,
- le distéaryldisulfure,
- le diallyldisulfure,
- le dicyclohexyldisulfure,
- le diphényldisulfure,
- le dibenzyldisulfure,
- le dibenzoyldisulfure,
- le dithiopyridine,
- l'acide dithioglycolique.

**26.** Procédé selon l'une des revendications 1 à 25 **caractérisé par le fait que** le catalyseur de couplage est un catalyseur comprenant au moins un élément métallique choisi parmi la 4$^{ème}$ et 5$^{ème}$ période des groupes IIIA, IVA, VA, VIA, VIIA, VIII, IB et IIB de la classification périodique des éléments.

**27.** Procédé selon la revendication 26 **caractérisé par le fait que** le catalyseur de couplage est un catalyseur comprenant au moins l'un des éléments métalliques suivants ; cuivre, fer, cobalt, nickel, palladium, platine.

**28.** Procédé selon l'une des revendications 26 et 27 **caractérisé par le fait que** le catalyseur de couplage est un catalyseur dans lequel l'élément métallique est également apporté sous forme de métal zéro ou d'un dérivé inorganique tel qu'un oxyde ou un hydroxyde ou d'un sel minéral de préférence, nitrate, sulfate, oxysulfate, halogénure, oxyhalogénure, silicate, carbonate, ou à un dérivé organique de préférence, cyanure, oxalate, acétylacétonate ; alcoolate et encore plus préférentiellement méthylate ou éthylate ; carboxylate et encore plus préférentiellement acétate.

**29.** Procédé selon l'une des revendications 26 à 28 **caractérisé par le fait que** le catalyseur est le cuivre métal ou un composé organique ou inorganique du cuivre I ou du cuivre II.

**30.** Procédé selon la revendication 29 **caractérisé par le fait que** le composé du cuivre est choisi parmi le bromure cuivreux, le bromure cuivrique, l'iodure cuivreux, le chlorure cuivreux, le chlorure cuivrique, le carbonate de cuivre II basique, le nitrate cuivreux, le nitrate cuivrique, le sulfate cuivreux, le sulfate cuivrique, le sulfite cuivreux, l'oxyde cuivreux, l'acétate cuivreux, l'acétate cuivrique, le trifluorométhylsulfonate cuivrique, l'hydroxyde cuivrique, le méthylate de cuivre I, le méthylate de cuivre II, le méthylate chlorocuivrique de formule $ClCuOCH_3$.

**31.** Procédé selon l'une des revendications 26 à 31 **caractérisé par le fait que** la quantité de catalyseur mise en oeuvre exprimée par rapport au poids de sel de diazonium varie entre 0,1 % et 20 % en mol, de préférence, entre 1 et 10 %.

**32.** Procédé selon la revendication 1 **caractérisé par le fait que** la quantité de composé soufré est telle que le rapport entre le nombre de composé soufré et le nombre de moles de sel de diazonium varie entre 1 et 1,5.

**33.** Procédé selon l'une des revendications 1 à 32 **caractérisé par le fait que** la réaction de couplage est conduite en milieu aqueux : la quantité d'eau présente dans le milieu réactionnel représentant de 100 à 500 % du poids de l'amine aromatique.

**34.** Procédé selon l'une des revendications 1 à 33 **caractérisé par le fait que** la réaction de couplage est conduite en présence d'un solvant organique, de préférence, un acide carboxylique saturé et encore plus préférentiellement l'acide acétique.

**35.** Procédé selon l'une des revendications1 à 34 **caractérisé par le fait que** l'on ajoute le composé soufré de type

disulfure dans le sel de diazonium puis le catalyseur.

**36.** Procédé selon l'une des revendications 1 à 35 **caractérisé par le fait que** la réaction de couplage entre le sel de diazonium répondant préférentiellement à la formule (III) et le composé soufré répondant préférentiellement à la formule (IV) est conduite à une température variant entre 0°C et 120°C, de préférence, entre 80°C et 100°C.

**37.** Procédé selon l'une des revendications 1 à 36 **caractérisé par le fait que** l'on obtient, en fin de réaction, un composé qui répond préférentiellement à la formule (VI) :

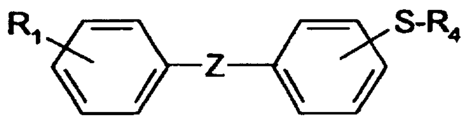

(VI)

dans ladite formule (VI), $R_1$, $R_4$, et Z ont la signification donnée dans les revendications 3 à 6,15 à 23.

**38.** Procédé selon la revendication 37 **caractérisé par le fait que** le composé de formule (VI) est le 4-chloro-4'-thiométhyldiphényléther.

**Patentansprüche**

**1.** Verfahren zur Herstellung eines aromatischen Thioesters vom Diphenyltyp, **dadurch gekennzeichnet, daß** man in wäßrigem Milieu ein Diazoniumsalz einer aromatischen Verbindung vom Diphenyltyp und eine Schwefelverbindung vom Disulfidtyp in Gegenwart einer wirksamen Menge eines Kupplungskatalysators reagieren läßt.

**2.** Verfahren nach Anspruch 1, **dadurch gekennzeichnet, daß** das Verfahren darin besteht, das Diazoniumsalz ausgehend von dem entsprechenden aromatischen Amin herzustellen, dann ohne Trennung die Reaktion mit der Schwefelverbindung durchzuführen.

**3.** Verfahren nach einem der Ansprüche 1 und 2, **dadurch gekennzeichnet, daß** das aromatische Ausgangsamin der allgemeinen Formel (I) entspricht

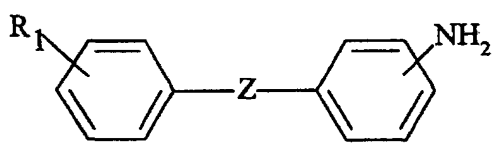

(I)

wobei in der Formel (I)

$R_1$ ein Wasserstoffatom oder einen Substituenten R darstellt, ausgewählt aus:

- einer linearen oder verzweigten Alkylgruppe mit 1 bis 6 Kohlenstoffatomen,
- einer linearen oder verzweigten Alkenylgruppe mit 2 bis 6 Kohlenstoffatomen,
- einer linearen oder verzweigten Halogenalkylgruppe mit vorzugsweise 1 bis 4 Kohlenstoffatomen und 1 bis 9 Halogenatomen,
- einer Cycloalkylgruppe mit 3 bis 7 Kohlenstoffatomen,
- einer Phenylgruppe,
- einer Hydroxylgruppe,

- einer $NO_2$-Gruppe,

- einer $R_3$-O-Alkoxygruppe oder einer $R_3$-S-Thioethergruppe, in der $R_3$ eine lineare oder verzweigte Alkylgruppe mit 1 bis 6 Kohlenstoffatomen oder eine Phenylgruppe darstellt,

- einer $-N-(R_2)_2$-Gruppe, in der die Gruppen $R_2$, identisch oder verschieden, Wasserstoff, eine lineare oder verzweigte Alkylgruppe mit 1 bis 6 Kohlenstoffatomen oder eine Phenylgruppe darstellen,

- einer $-NH-CO-R_2$-Gruppe, in der die Gruppe $R_2$ die zuvor angegebene Bedeutung hat,

- einer Carboxygruppe oder einem Derivat $R_2$-O-CO-, wobei die Gruppe $R_2$ die zuvor angegebene Bedeutung hat,

- einer Acyloxy- oder Aryloxy-Gruppe $R_3$CO-O-, in der die Gruppe $R_3$ die zuvor angegebene Bedeutung hat,

- einer Gruppe $B(OR_3)_2$, in der die Gruppe $R_3$ die zuvor angegebene Bedeutung hat,

- einem Halogenatom,

- einer $CF_3$-Gruppe,

- wobei zwei Gruppen R verbunden sein können und gemeinsam eine Alkylendioxygruppe mit 1 bis 4 Atomen in der Alkylengruppe bilden können,

Z darstellt:

. eine Valenzbindung

. eine Alkylen- oder Alkylidengruppe mit 1 bis 4 Kohlenstoffatomen

. eine Gruppe B, die ein Atom oder eine der folgenden Gruppen darstellen kann,
-O-, -CO-, -COO-, -OOC-, -OCOO-
-S-, -SO-, $-SO_2-$

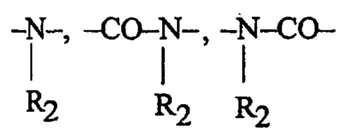

wobei in diesen Formeln $R_2$ ein Wasserstoffatom oder eine Alkylgruppe mit 1 bis 6 Kohlenstoffatomen oder Phenyl darstellt.

4. Verfahren nach Anspruch 3, **dadurch gekennzeichnet, daß** das aromatische Ausgangsamin der allgemeinen Formel (I) entspricht, in der:

$R_1$ ein Wasserstoffatom oder einen Substituenten R darstellt, ausgewählt aus:

- einer linearen oder verzweigten Alkylgruppe mit vorzugsweise 1 bis 4 Kohlenstoffatomen,

- einer linearen oder verzweigten Alkenylgruppe mit 2 bis 4 Kohlenstoffatomen,

- einer Cyclohexylgruppe,

- einer $R_3$-O-Alkoxygruppe oder einer $R_3$-S-Thioethergruppe, in der $R_3$ eine lineare oder verzweigte Alkylgruppe mit 1 bis 4 Kohlenstoffatomen oder eine Phenylgruppe darstellt,

- einer $-N-(R_2)_2$-Gruppe, in der die Gruppen $R_2$, identisch oder verschieden, ein Wasserstoffatom, eine lineare oder verzweigte Alkylgruppe mit 1 bis 4 Kohlenstoffatomen oder eine Phenylgruppe darstellen,

- einer $-NH-CO-R_2$-Gruppe, in der die Gruppe $R_2$ die zuvor angegebene Bedeutung hat,

- einer Carboxygruppe oder einem Derivat $R_2-O-CO-$, wobei die Gruppe $R_2$ die zuvor angegebene Bedeutung hat,

- einer Acyloxy- oder Aryloxygruppe $R_3CO-O-$, in der die Gruppe $R_3$ die zuvor angegebene Bedeutung hat,

- einer Gruppe $B(OR_3)_2$, in der die Gruppe $R_3$ die zuvor angegebene Bedeutung hat,

- einem Fluoratom,

- wobei zwei Gruppen R verbunden sein können und gemeinsam eine Methylendioxy- oder Ethylendioxygruppe bilden können,

Z darstellt:

. eine Valenzbindung,

. eine Methylen- oder Isopropylidengruppe,

. eine Gruppe B, die ein Atom oder eine der folgenden Gruppen darstellen kann:
-O-, -CO-, -COO-, -OOC-, -OCOO-
-S-, -SO-, $-SO_2-$

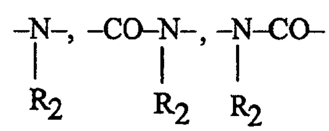

wobei in diesen Formeln $R_2$ ein Wasserstoffatom oder eine Alkylgruppe mit 1 bis 6 Kohlenstoffatomen oder Phenyl darstellt.

5. Verfahren nach Anspruch 4, **dadurch gekennzeichnet, daß** das aromatische Ausgangsamin der allgemeinen Formel (I) entspricht, in der die Gruppe R ein Fluor-, Chlor- oder Bromatom oder eine Perfluoralkylgruppe; eine Hydroxylgruppe; eine Alkyl- oder Alkoxygruppe mit 1 bis 4 Kohlenstoffatomen; eine Aminogruppe oder eine mit einer oder zwei Alkylgruppen mit 1 bis 4 Kohlenstoffatomen substituierte Aminogruppe darstellt.

6. Verfahren nach einem der Ansprüche 1 bis 5, **dadurch gekennzeichnet, daß** das aromatische Ausgangsamin der allgemeinen Formel (I) entspricht, in der $R_1$ ein Fluor- oder Chloratom darstellt und Z ein Sauerstoffatom darstellt.

7. Verfahren nach einem der Ansprüche 1 bis 6, **dadurch gekennzeichnet, daß** man das Halohydrat des aromatischen Amins vom Diphenyltyp, welches vorzugsweise der Formel (I) entspricht, herstellt, indem man letzteres mit einer Wasserstoffsäure, vorzugsweise Chlorwasserstoff- oder Bromwasserstoffsäure, reagieren läßt.

8. Verfahren nach Anspruch 7, **dadurch gekennzeichnet, daß** die eingesetzte Säuremenge derart ist, daß das Molverhältnis zwischen der Anzahl der $H^+$-Ionen und der Anzahl der Mol an Ausgangsverbindung zwischen 2,0 und 2,5, vorzugsweise zwischen 2,0 und 2,2, variiert

9. Verfahren nach einem der Ansprüche 7 und 8, **dadurch gekennzeichnet, daß** die Reaktion zwischen dem aromatischen Amin und der Wasserstoffsäure bei einer Temperatur zwischen 50°C und 100 °C durchgeführt wird.

10. Verfahren nach einem der Ansprüche 1 bis 9, **dadurch gekennzeichnet, daß** man das Diazoniumsalz durch Reaktion des aromatischen Amins vom Diphenyltyp in Form des Halohydrats mit einem Diazotierungsreagenz,

welches jede beliebige $NO^+$-Quelle sein kann, durchfährt.

**11.** Verfahren nach Anspruch 10, **dadurch gekennzeichnet, daß** das Diazotierungsreagenz jede beliebige $NO^+$-Quelle ist, vorzugsweise Stickstoffoxid NO, zusammen mit einem Oxidationsmittel; Stickstoffdioxid $NO_2$; Distickstofftrioxid $N_2O_3$; Distickstofftetroxid $N_2O_4$; Salpetrige Säure; Nitrosylsulfat oder ein Salpetriges Salz, vorzugsweise ein Alkalymetallsalz, vorzugsweise ein Natriumsalz, oder ein Alkylnitrit.

**12.** Verfahren nach Anspruch 11, **dadurch gekennzeichnet, daß** die eingesetzte Diazotierungsreagenzmenge, berechnet als Molverhältnis aromatisches Amin/Diazotierungsreagenz in Form von $NO^+$, zwischen 100 % und 120 % liegt.

**13.** Verfahren nach einem der Ansprüche 10 bis 12, **dadurch gekennzeichnet, daß** die Temperatur der Diazotierungsreaktion zwischen -10 °C und 20 °C, vorzugsweise zwischen 0 und 10 °C, liegt.

**14.** Verfahren nach einem der Ansprüche 10 bis 13, **dadurch gekennzeichnet, daß** das erhaltene Diazoniumsalz vorzugsweise der Formel (III) entspricht:

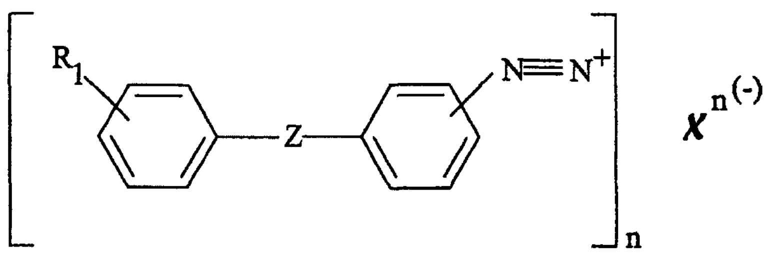

(III)

wobei in der Formel (III):

- X ein Halogenatom X, vorzugsweise ein Chlor- oder Bromatom, eine Gruppe $HSO_4$- oder eine Gruppe $SO_4^{2-}$ darstellt,
- $R_1$ und Z die zuvor in einem der Ansprüche 3 bis 6 angegebene Bedeutung haben,
- n gleich 1 oder 2 ist.

**15.** Verfahren nach einem der Ansprüche 1 und 2, **dadurch gekennzeichnet, daß** die zur Reaktion gebrachte Schwefelverbindung der folgenden Formel (IV) entspricht:

$$R_4\text{-S-S-}R_5 \qquad \text{(IV)}$$

wobei in der Formel (IV):

- $R_4$ und $R_5$, identisch oder verschieden, eine Kohlenwasserstoffgruppe mit 1 bis 24 Kohlenstoffatomen, die eine gesättigte oder ungesättigte, lineare oder verzweigte acyclische aliphatische Gruppe sein kann; eine monocyclische oder polycyclische, gesättigte, ungesättigte oder aromatische, carbocyclische oder heterocyclische Gruppe; oder eine gesättigte oder ungesättigte, lineare oder verzweigte aliphatische Gruppe, die einen ringförmigen Substituenten trägt, darstellen.

**16.** Verfahren nach Anspruch 15, **dadurch gekennzeichnet, daß** die Schwefelverbindung der allgemeinen Formel (IV) entspricht, in der $R_4$ und $R_5$ eine lineare oder verzweigte, gesättigte oder ungesättigte, acyclische aliphatische Gruppe mit vorzugsweise 1 bis 24 Kohlenstoffen darstellt, die gesättigt ist oder ein oder mehrere ungesättigte Bindungen in der Kette, im allgemeinen 1 bis 3 ungesättigte Bindungen enthält, die einfache Doppelbindungen oder konjugierte Bindungen oder Dreifachbindungen sein können.

**17.** Verfahren nach einem der Ansprüche 15 und 16, **dadurch gekennzeichnet, daß** die Schwefelverbindung der allgemeinen Formel (IV), in der $R_4$ und $R_5$ eine lineare oder verzweigte Alkyl-, Alkenyl oder Alkadienylgruppe mit vorzugsweise 1 bis 12 Kohlenstoffatomen darstellt, wobei die Kohlenwasserstoffkette gegebenenfalls:

- unterbrochen sein kann durch ein Atom oder eine funktionelle Gruppe B, wie in Anspruch 3 definiert,
- und/oder Träger einer der folgenden Substituenten sein kann:
  $-OH$, $-COR_3$, $-COOR_2$, $-CHO$, $-CN$, $-NO_2$, $-X$, $-CF_3$

wobei in diesen Formeln die Gruppen $R_2$, identisch oder verschieden, und die Gruppe $R_3$ die zuvor angegebenen Bedeutungen haben.

**18.** Verfahren nach einem der Ansprüche 15 bis 17, **dadurch gekennzeichnet, daß** die Schwefelverbindung der allgemeinen Formel (IV) entspricht, in der $R_4$ und $R_5$ eine lineare oder verzweigte Halogenalkylgruppe mit vorzugsweise 1 bis 12 Kohlenstoffatomen, besonders bevorzugt 1 bis 4 Kohlenstoffatomen, und 3 bis 25 Halogenatomen darstellt.

**19.** Verfahren nach einem der Ansprüche 15 bis 18, **dadurch gekennzeichnet, daß** die Schwefelverbindung der allgemeinen Formel (IV) entspricht, in der $R_4$ und $R_5$ eine lineare oder verzweigte, gesättigte oder ungesättigte, acyclische aliphatische Gruppe, die Träger eines ringförmigen Substituenten, vorzugsweise eines Benzolrings, ist, darstellen, wobei die acyclische aliphatische Gruppe mit dem Ring über eine Valenzbindung oder über eine der Gruppen B, wie in Anspruch 3 definiert, verbunden sein kann.

**20.** Verfahren nach Anspruch 15, **dadurch gekennzeichnet, daß** die Schwefelverbindung der allgemeinen Formel (IV) entspricht, in der $R_4$ und $R_5$ darstellen:

- eine monocyclische carbocyclische Gruppe, die gesättigt ist oder ein oder zwei ungesättigte Bindungen im Ring enthält, wobei die Anzahl der Kohlenstoffatome in dem Ring zwischen 3 und 8 Kohlenstoffatomen variiert und vorzugsweise 5 oder 6 beträgt,
- eine polycyclische, vorzugsweise bicyclische, carbocyclische Gruppe, wobei die Anzahl der Kohlenstoffatome in jedem Ring zwischen 3 und 6 variiert, wobei die Gesamtanzahl an Kohlenstoffatomen vorzugsweise 7 beträgt.

**21.** Verfahren nach Anspruch 15, **dadurch gekennzeichnet, daß** die Schwefelverbindung der allgemeinen Formel (IV) entspricht, in der die Gruppen $R_4$ und $R_5$ eine aromatische carbocyclische Gruppe, vorzugsweise eine Benzolgruppe oder eine Verkettung bzw. Aneinanderreihung von 2 oder 3 Benzolkernen, die über Atome oder Gruppen B, wie in Anspruch 3 definiert, getrennt sind.

**22.** Verfahren nach Anspruch 15, **dadurch gekennzeichnet, daß** die Schwefelverbindung der allgemeinen Formel (IV) entspricht, in der $R_4$ und $R_5$ darstellen:

- eine aromatische heterocyclische Gruppe mit 5 oder 6 Atomen im Ring, hierunter ein oder zwei Heteroatome,
- eine carbocyclische oder heterocyclische aromatische polycyclische Gruppe.

**23.** Verfahren nach einem der Ansprüche 15 bis 22, **dadurch gekennzeichnet, daß** die Schwefelverbindung der allgemeinen Formel (IV) entspricht, in der $R_4$ und $R_5$ eine lineare oder verzweigte Alkylgruppe mit 1 bis 4 Kohlenstoffatomen, eine 2-Carboxyethyl-, eine Cyclohexyl-, eine Phenyl-, eine Benzyl-, eine Benzoyl- oder eine Pyridylgruppe darstellen.

**24.** Verfahren nach einem der Ansprüche 15 bis 23, **dadurch gekennzeichnet, daß** die Schwefelverbindung der allgemeinen Formel (IV) entspricht, in der $R_4$ und $R_5$ identisch sind.

**25.** Verfahren nach Anspruch 15, **dadurch gekennzeichnet, daß** die Schwefelverbindung der allgemeinen Formel (IV) eine Verbindung vom Disulfidtyp ist, ausgewählt aus:

- Dimethyldisulfid,

- Diethyldisulfid,
- n-Propyldisulfid,
- Diisopropyldisulfid,
- Di-n-butyldisulfid,
- Düsobutyldisulfid,
- Di-sek.-butyldisulfid,
- Di-tert.-butyldisulfid,
- Diisoamyldisulfid,
- Di-n-hexyldisulfid,
- Di-tert.-heptyldisulfid,
- Di-n-undecyldisulfid,
- Distearyldisulfid,
- Diallyldisulfid,
- Dicyclohexyldisulfid,
- Diphenyldisulfid,
- Dibenzyldisulfid,
- Dibenzoyldisulfid,
- Dithiopyridin,
- Dithioglykolsäure.

**26.** Verfahren nach einem der Ansprüche 1 bis 25, **dadurch gekennzeichnet, daß** der Kupplungskatalysator ein Katalysator mit mindestens einem metallischen Element ist, ausgewählt aus der vierten und fünften Periode der Gruppen IIIA, IVA, VA, VIA, VIIA, VIII, IB und IIB des Periodensystems der Elemente.

**27.** Verfahren nach Anspruch 26, **dadurch gekennzeichnet, daß** der Kupplungskatalysator ein Katalysator mit mindestens einem der folgenden metallischen Elemente ist: Kupfer, Eisen, Cobalt, Nickel, Palladium und Platin.

**28.** Verfahren nach einem der Ansprüche 26 und 27, **dadurch gekennzeichnet, daß** der Kupplungskatalysator ein Katalysator ist, bei dem das metallische Element gleichermaßen in Form eines Metalls Null oder einer anorganischen Verbindung, wie einem Oxid oder Hydroxid oder einem anorganischen Salz, vorzugsweise Nitrat, Sulfat, Oxysulfat, Halogenid, Oxyhalogenid, Silikat oder Carbonat, oder einem organischen Derivat, vorzugsweise Cyanid, Oxalat, Acetylacetonat, einem Alkoholat, besonders bevorzugt einem Methylat oder Ethylat, einem Carboxylat oder bevorzugt einem Acetat bereitgestellt werden kann.

**29.** Verfahren nach einem der Ansprüche 26 bis 28, **dadurch gekennzeichnet, daß** der Katalysator ein Kupfermetall ist oder eine organische oder anorganische Verbindung von Kupfer (I) oder Kupfer (II).

**30.** Verfahren nach Anspruch 29, **dadurch gekennzeichnet, daß** die Kupferverbindung ausgewählt ist aus Kupfer(I)bromid, Kupfer(II)bromid, Kupfer(I)iodid, Kupfer(I)chlorid; basischem Kupfer(II)carbonat, Kupfer(I)-nitrat, Kupfer(II)nitrat, Kupfer(I)sulfat, Kupfer(II)sulfat, Kupfer(I)sulfit, Kupfer(I)oxid, Kupfer(I)acetat, Kupfer(II)acetat, Kupfer(II)trifluormethylsulfonat, Kupfer(II)hydroxid, Kupfer(I)methylat, Kupfer(II)methylat, Chlorokupfer(II)methylat der Formel $ClCuOCH_3$.

**31.** Verfahren nach einem der Ansprüche 26 bis 31, **dadurch gekennzeichnet, daß** die eingesetzte Katalysatormenge, berechnet in bezug auf das Gewicht des Diazoniumsalzes, zwischen 0,1 Mol-% und 20 Mol-%, vorzugsweise zwischen 1 und 10 Mol-%, variiert.

**32.** Verfahren nach Anspruch 1, **dadurch gekennzeichnet, daß** die Menge an Schwefelverbindung derart ist, daß das Verhältnis zwischen der Anzahl der Mol der Schwefelverbindung und der Anzahl der Mol des Diazoniumsalzes zwischen 1 und 1,5 variiert.

**33.** Verfahren nach einem der Ansprüche 1 bis 32, **dadurch gekennzeichnet, daß** die Kupplungsreaktion in wäßrigem Milieu durchgeführt wird, wobei die in dem Reaktionsmilieu vorhandene Wassermenge 100 bis 500 Gew.-% des aromatischen Amins beträgt.

**34.** Verfahren nach einem der Ansprüche 1 bis 33, **dadurch gekennzeichnet, daß** die Kupplungsreaktion in Gegenwart eines organischen Lösemittels, vorzugsweise einer gesättigten Carbonsäure, insbesondere Essigsäure, durchgeführt wird.

**35.** Verfahren nach einem der Ansprüche 1 bis 34, **dadurch gekennzeichnet, daß** man die Schwefelverbindung vom Disulfidtyp zu dem Diazoniumsalz hinzugibt, dann den Katalysator.

**36.** Verfahren nach einem der Ansprüche 1 bis 35, **dadurch gekennzeichnet, daß** die Kupplungsreaktion zwischen dem Diazoniumsalz, vorzugsweise gemäß der Formel (III), und der Schwefelverbindung, vorzugsweise gemäß der Formel (IV), bei einer Temperatur von 0 °C bis 120 °C, vorzugsweise 80 °C bis 100 °C, durchgeführt wird.

**37.** Verfahren nach einem der Ansprüche 1 bis 36, **dadurch gekennzeichnet, daß** man am Ende der Reaktion eine Verbindung erhält, die vorzugsweise der Formel (VI):

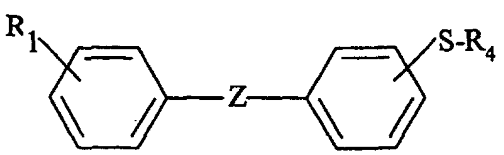

(VI)

entspricht, wobei in der Formel (VI) $R_1$, $R_4$ und Z die zuvor in den Ansprüchen 3 bis 6 und 15 bis 23 angegebene Bedeutung haben.

**38.** Verfahren nach Anspruch 37, **dadurch gekennzeichnet, daß** die Verbindung der Formel (VI) 4-Chlor-4'-thiomethyldiphenylether ist.

## Claims

**1.** A process for preparing a biphenyl type aromatic thioether, **characterized in that** a diazonium salt of a biphenyl type aromatic compound is reacted with a disulphide type sulphur-containing compound in an aqueous medium in the presence of an effective quantity of a coupling catalyst.

**2.** A process according to claim 1, **characterized in that** it consists of preparing the diazonium salt from the corresponding aromatic amine then, without separation, carrying out the reaction with the sulphur-containing compound.

**3.** A process according to claim 1 or claim 2, **characterized in that** the aromatic starting amine has general formula (I):

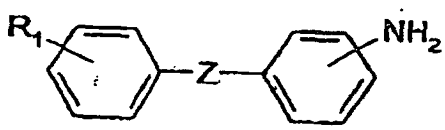

(I)

in which formula (I):

- $R_1$ represents a hydrogen atom or a substituent R selected from:
  - a linear or branched alkyl group containing 1 to 6 carbon atoms;
  - a linear or branched alkenyl group containing 2 to 6 carbon atoms;
  - a linear or branched halogenoalkyl group, preferably containing 1. to 4 carbon atoms, and 1 to 9 halogen atoms;
  - a cycloalkyl group containing 3 to 7 carbon atoms;
  - a phenyl group;
  - a hydroxyl group;
  - a $NO_2$ group;
  - a $R_3$-O- alkoxy group or $R_3$-S- thioether group where $R_3$ represents a linear or branched alkyl group containing 1 to 6 carbon atoms, or a phenyl group;
  - a -N-$(R_2)_2$ group where groups $R_2$, which may be identical or different, represent a hydrogen atom, a linear or branched alkyl group containing 1 to 6 carbon atoms, or a phenyl group;
  - a -NH-CO-$R_2$ group where $R_2$ has the meaning given above;
  - a carboxyl group or $R_2$-O-CO- derivative, where group $R_2$ has the meaning given above;
  - an acyloxy or aroyloxy group $R_3$-CO-O-, where group $R_3$ has the meaning given above;
  - a $B(OR_3)_2$ group, where group $R_3$ has the meaning given above;
  - a halogen atom;
  - a $CF_3$ group;
  - two groups R can together form an alkylenedioxy group containing 1 to 4 atoms in the alkylene group;

- Z represents:
  - a covalent bond;
  - an alkylene or alkylidene group containing 1 to 4 carbon atoms;
  - a group B which may be one of the following atoms or groups:
    -O-, -CO-, -COO-, -OOC-, -OCOO-
    -S-, -SO-, -$SO_2$-,

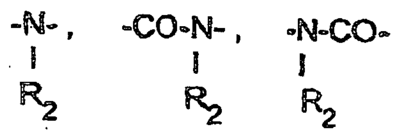

in which formulae, $R_2$ represents a hydrogen atom or an alkyl group containing 1 to 6 carbon atoms, or a phenyl group.

**4.** A process according to claim 3, **characterized in that** the aromatic starting amine has general formula (I) in which:

- $R_1$ represents a hydrogen atom or any other group R selected from:
  - a linear or branched alkyl group, preferably containing 1 to 4 carbon atoms;
  - a linear or branched alkenyl group containing 2 to 4 carbon atoms;
  - a cyclohexyl group;
  - a $R_3$-O- alkoxy group or $R_3$-S- thioether group where $R_3$ represents a linear or branched alkyl group

containing 1 to 6 carbon atoms, preferably 1 to 4 carbon atoms, or a phenyl group;

  - a $-N-(R_2)_2$ group where groups $R_2$, which may be identical or different, represent a hydrogen atom, a linear or branched alkyl group containing 1 to 4 carbon atoms, or a phenyl group;
  - a $-NH-CO-R_2$ group where $R_2$ has the meaning given above;
  - a carboxyl group or $R_2$-O-CO- derivative, where group $R_2$ has the meaning given above;
  - an acyloxy or aroyloxy group $R_3$-CO-O-, where group $R_3$ has the meaning given above;
  - a $B(OR_3)_2$ group, where group $R_3$ has the meaning given above;
  - a fluorine atom;
  - two groups R can together form a methylenedioxy or ethylenedioxy group;

- Z represents:

  - a covalent bond;
  - an methylene or isopropylidene group;
  - a group B which may be one of the following atoms or groups:
    -O-, -CO-, -COO-, -OOC-, -OCOO-
    -S-, -SO-, $-SO_2-$,

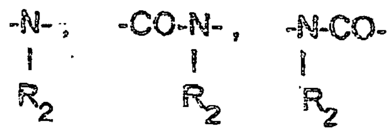

in which formulae, $R_2$ represents a hydrogen atom or an alkyl group containing 1 to 6 carbon atoms, or a phenyl group.

**5.** A process according to claim 4, **characterized in that** the aromatic starting amine has general formula (I) in which group R is a fluorine, chlorine or bromine atom, or a perfluoroalkyl group; a hydroxyl group; an alkyl or alkoxy group containing 1 to 4 carbon atoms; an amino group or an amino group substituted by one or two alkyl groups containing 1 to 4 carbon atoms.

**6.** A process according to any one of claims 1 to 5, **characterized in that** the aromatic starting amine has general formula (I) where $R_1$ represents a fluorine atom or chlorine atom and Z represents an oxygen atom.

**7.** A process according to any one of claims 1 to 6, **characterized in that** the halohydrate of the biphenyl type aromatic amine preferably with formula (I) is prepared by reacting said amine with a hydrogen acid, preferably hydrochloric acid or hydrobromic acid.

**8.** A process according to claim 7, **characterized in that** the quantity of acid used is such that the mole ratio between the number of $H^+$ ions and the number of moles of substrate is in the range 2.0 to 2.5, preferably in the range 2.0 to 2.2.

**9.** A process according to claim 7 or claim 8, **characterized in that** the reaction between the aromatic amine and the hydrogen acid is carried out at a temperature in the range 50°C to 100°C.

**10.** A process according to any one of claims 1 to 9, **characterized in that** the diazonium salt is prepared by reacting the biphenyl type aromatic amine in the halohydrate form with a diazotisation reactant that is any source of $NO^+$.

**11.** A process according to claim 10, **characterized in that** the diazotisation reactant is any source of $NO^+$, preferably nitric oxide NO associated with an oxidising agent; nitrogen dioxide $NO_2$, nitrogen trioxide $N_2O_3$, nitrogen tetroxide $N_2O_4$; nitrous acid; nitrosyl sulphate or a nitrous salt, preferably an alkali metal salt, more preferably sodium; or an alkyl nitrite.

**12.** A process according to claim 11, **characterized in that** the quantity of diazotisation reactant used, expressed as the mole ratio of the aromatic amine/diazotisation reactant defined as $NO^+$, is in the range 100% to 120%.

**13.** A process according to any one of claims 10 to 12, **characterized in that** the temperature of the diazotisation reaction is in the range -10°C to 20°C, preferably in the range 0°C to 10°C.

**14.** A process according to any one of claims 10 to 13, **characterized in that** the diazonium salt obtained preferably has formula (III):

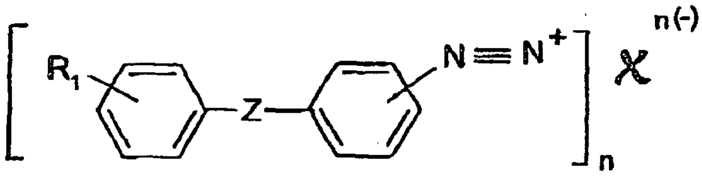

(III)

in which formula (III):

- X represents a halogen atom X, preferably a chlorine or bromine atom, a $HSO_4^-$ group or a $SO_4^=$ group;
- $R_1$ and Z have the meanings defined in any one of claims 3 to 6;
- n equals 1 or 2.

**15.** A process according to claim 1 or claim 2, **characterized in that** the sulphur-containing compound used has the following formula (IV):

$$R_4\text{-S-S-}R_5 \qquad (IV)$$

in said formula (IV):

- $R_4$ and $R_5$, which may be identical or different, represent a hydrocarbon group containing 1 to 24 carbon atoms, which can be a saturated or unsaturated, linear or branched acyclic aliphatic group; a saturated, unsaturated or aromatic, monocyclic or polycyclic carbocyclic or heterocyclic group; or a linear or branched, saturated or unsaturated aliphatic group carrying a cyclic substituent.

**16.** A process according to claim 15, **characterized in that** the sulphur-containing compound has general formula (IV) where $R_4$ and $R_5$ represent a saturated or unsaturated, linear or branched acyclic aliphatic group preferably containing 1 to 24 carbon atoms, saturated or comprising one or more unsaturated bonds in the chain, generally 1 to 3 unsaturated bonds which may be simple or conjugated double bonds, or triple bonds.

**17.** A process according to claim 15 or claim 16, **characterized in that** the sulphur-containing compound has general formula (IV) where $R_4$ and $R_5$ represent a linear or branched alkyl, alkenyl or alkadienyl group preferably containing 1 to 12 carbon atoms; the hydrocarbon chain can optionally be:

- interrupted by an atom or a functional group B as defined in claim 3;
- and/or carry one of the following substituents:
  - OH, $-COR_3$, $-COOR_2$, -CHO, -CN, $-NO_2$, -X, $-CF_3$;

in which formulae, groups $R_2$, may be identical or different, and group $R_3$ have the meanings defined above.

**18.** A process according to any one of claims 15 to 17, **characterized in that** the sulphur-containing compound has general formula (IV) where $R_4$ and $R_5$ represent a linear or branched halogenoalkyl group preferably containing 1 to 12 carbon atoms and more preferably 1 to 4 carbon atoms, and 3 to 25 halogen atoms.

**19.** A process according to any one of claims 15 to 18, **characterized in that** the sulphur-containing compound has general formula (IV) where $R_4$ and $R_5$ represent a saturated or unsaturated, linear or branched acyclic aliphatic group carrying a cyclic substituent, preferably a benzene ring: the acyclic aliphatic group may be bonded to the ring via a covalent bond or by a group B as defined in claim 3.

**20.** A process according to claim 15, **characterized in that** the sulphur-containing compound has general formula (IV) in which $R_4$ and $R_5$ represent:

- a monocyclic carbocyclic group that is saturated or contains 1 or 2 unsaturated bonds in the cycle; the number of carbon atoms in the cycle may be in the range 3 to 8 carbon atoms, preferably 5 or 6;
- a polycyclic carbocyclic group, preferably bicyclic; the number of carbon atoms in each cycle is in the range 3 to 6: the total number of carbon atoms preferably being equal to 7.

**21.** A process according to claim 15, **characterized in that** the sulphur-containing compound has general formula (IV) in which groups $R_4$ and $R_5$ represent an aromatic carbocyclic group, in particular a benzene ring or a concatenation of 2 or 3 benzene rings separated by atoms or groups B as defined in claim 3.

**22.** A process according to claim 15, **characterized in that** the sulphur-containing compound has general formula (IV) where $R_4$ and $R_5$ represent:

- an aromatic heterocyclic group containing 5 or 6 atoms in the ring, 1 or 2 of which are heteroatoms;
- a polycyclic aromatic carbocyclic or heterocyclic group.

**23.** A process according to any one of claims 15 to 22, **characterized in that** the sulphur-containing compound has general formula (IV) where groups $R_4$ and $R_5$ represent a linear or branched alkyl group containing 1 to 4 carbon atoms, or a 2-carboxyethyl, cyclohexyl, phenyl, benzyl, benzoyl or pyridyl group.

**24.** A process according to claim 15 to 23, **characterized in that** the sulphur-containing compound has general formula (IV) in which $R_4$ and $R_5$ are identical.

**25.** A process according to claim 15, **characterized in that** the sulphur-containing compound having general formula (IV) is a disulphide type compound selected from:

- dimethyldisulphide;
- diethyldisulphide;
- di-n-propyldisulphide;
- diisopropyldisulphide;
- di-n-butyldisulphide;
- diisobutyldisulphide;
- di-sec-butyldisulphide;
- di-tert-butyldisulphide;
- diisoamyldisulphide;
- di-n-hexyldisulphide;
- di-tert-heptyldisulphide;
- di-n-undecyldisulphide;
- distearyldisulphide;
- diallyldisulphide;
- dicyclohexyldisulphide;
- diphenyldisulphide;
- dibenzyldisulphide;
- dibenzoyldisulphide;
- dithiopyridine;
- dithioglycolic acid.

**26.** A process according to any one of claims 1 to 25, **characterized in that** the coupling catalyst is a catalyst comprising at least one metallic element selected from the $4^{th}$ and $5^{th}$ period of groups IIIA, IVA, VA, VIA, VIIA, VIII, IB and IIB of the periodic table.

**27.** A process according to claim 26, **characterized in that** the coupling catalyst is a catalyst comprising at least one of the following metallic elements: copper, iron, cobalt, nickel, palladium and platinum.

**28.** A process according to claim 26 or claim 27, **characterized in that** the coupling catalyst is a catalyst in which the metallic element is supplied in the form of a zero metal or an inorganic derivative such as an oxide or hydroxide

or a mineral salt, preferably a nitrate, sulphate, oxysulphate, halide, oxyhalide, silicate, carbonate or an organic derivative, preferably a cyanide, oxalate or acetylacetonate; an alcoholate, more preferably a methylate or ethylate; or a carboxylate, more preferably an acetate.

**29.** A process according to any one of claims 26 to 28, **characterized in that** the catalyst is copper metal or an organic or inorganic copper I or copper II compound.

**30.** A process according to claim 29, **characterized in that** the copper compound is selected from cuprous bromide, cupric bromide, cuprous iodide, cuprous chloride, cupric chloride, basic copper II carbonate, cuprous nitrate, cupric nitrate, cuprous sulphate, cupric sulphate, cuprous sulphite, cuprous oxide, cuprous acetate, cupric acetate, cupric trifluoromethylsulphonate, cupric hydroxide, copper I methylate, copper II methylate, and chlorocupric methylate with formula $ClCuOCH_3$.

**31.** A process according to any one of claims 26 to 31, **characterized in that** the quantity of catalyst used, expressed as the ratio of the weight of the diazonium salt, is in the range 0.1% to 20 mole %, preferably 1% to 10%.

**32.** A process according to claim 1, **characterized in that** the quantity of sulphur-containing compound is such that the ratio between the number of moles of sulphur-containing compound and the number of moles of diazonium salt is in the range 1 to 1.5.

**33.** A process according to any one of claims 1 to 32, **characterized in that** the coupling reaction is carried out in an aqueous medium: the quantity of water present in the reaction medium represents 100% to 500% by weight of the aromatic amine.

**34.** A process according to any one of claims 1 to 33, **characterized in that** the coupling reaction is carried out in the presence of an organic solvent, preferably a saturated carboxylic acid, more preferably acetic acid.

**35.** A process according to any one of claims 1 to 34, **characterized in that** the disulphide type sulphur-containing compound is added to the diazonium salt followed by the catalyst.

**36.** A process according to any one of claims 1 to 35, **characterized in that** the coupling reaction between the diazonium salt preferably with formula (III) and the sulphur-containing compound preferably with formula (IV) is carried out at a temperature in the range 0°C to 120°C, preferably in the range 80°C to 100°C.

**37.** A process according to any one of claims 1 to 36, **characterized in that** at the end of the reaction, a compound is obtained that preferably has the following formula (VI):

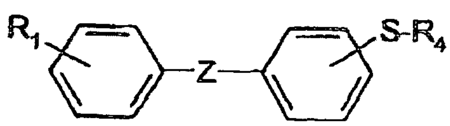

(VI)

in which formula (VI), $R_1$, $R_4$ and Z have the meanings defined in claims 3 to 6, and 15 to 23.

**38.** A process according to claim 37, **characterized in that** the compound with formula (VI) is 4-chloro-4'-thiomethyl-diphenylether.